# EUROPÄISCHE PATENTSCHRIFT

(11) **EP 0 782 412 B1**
(45) Veröffentlichungstag und Bekanntmachung des Hinweises auf die Patenterteilung: **13.12.2000**
(21) Anmeldenummer: 95932750.3
(22) Anmeldetag: 14.09.1995
(51) Int. Cl.: A61B 17/28

(54) **CHIRURGISCHES INSTRUMENT**
SURGICAL INSTRUMENT
INSTRUMENT DE CHIRURGIE

(30) Priorität: 20.09.1994 DE 4433403; 21.01.1995 DE 19501752
(43) Veröffentlichungstag der Anmeldung: 09.07.1997
(73) Patentinhaber: Koscher, Stephan, 78549 Spaichingen (DE); Würtz, Johann, 78549 Spaichingen (DE)
(72) Erfinder: Koscher, Stephan, 78549 Spaichingen (DE); Würtz, Johann, 78549 Spaichingen (DE)
(74) Vertreter: Weiss, Peter, Dr. rer.nat.
(86) Internationale Anmeldenummer: EP9503622
(87) Internationale Veröffentlichungsnummer: WO9609008

(56) Entgegenhaltungen:
- EP-A- 0 513 471
- CH-A- 243 732
- DE-A- 3 509 212
- GB-A- 1 441 608
- US-A- 1 577 880
- US-A- 2 069 636
- US-A- 2 725 629

## Beschreibung

Die Erfindung betrifft ein chirurgisches Instrument insbesondere für die Endoskopie mit einem Außenrohr, in dem ein Zug- oder Druckelement zum Betätigen von zumindest einem Maulteil geführt ist, das um eine Achse dreht.

Aus den US 2 069 636 und US 2 725 629 sind beispielsweise Scheren bekannt, bei denen die Klingen auswechselbar ausgestaltet sind.

Chirurgische Instrumente der o.g. Art sind ebenfalls in vielfältiger Form und Ausführung bekannt und auf dem Markt, siehe zum Beispiel EP-A-0 513 471. Mit den entsprechenden Maulteilen kann geschnitten, geklemmt, abgezwickt, geschert od.dgl. werden. Sie dienen in der Regel dazu, um bestimmte chirurgische Eingriffe bei Mensch oder Tier durchzuführen. Insbesondere durch die Endoskopie haben diese Instrumente und ihre Ausgestaltung wesentlich an Bedeutung gewonnen.

Als Beispiel wird hier noch auf ein chirurgisches Instrument entsprechend der DE-A-43 32 497.5 verwiesen. Dort sind die Maulteile über ein entsprechendes Zug- bzw. Druckelement mit Betätigungsorganen, beispielsweise Scherenschenkeln, verbunden. Bei Betätigung der Scherenschenkel erfolgt ein Öffnen und Schließen der Maulteile. Dabei können entweder beide Maulteile beweglich sein oder ein Maulteil feststehen und das andere Maulteil bewegt werden. Sobald die Maulteile abgenutzt sind, wird das Instrument weggeworfen.

Der vorliegenden Erfindung liegt die Aufgabe zugrunde, ein chirurgisches Instrument zu schaffen, welches immer wieder verwendbar ist, und bei dem ein Verschleiß der Maulteile kein Problem darstellt. Ferner sollte die Möglichkeit der Sterilisierung des Instruments erleichtert sein.

Zur Lösung dieser Aufgabe führt, daß das Maulteil über eine Aufnahme mit der Achse verbunden und das Maulteil gegenüber der Aufnahme lösbar festgelegt ist, während andernends des Maulteils die Aufnahme gelenkig mit dem Zug- oder Druckelement verbunden ist.

Grundlegender Gedanke der vorliegenden Erfindung ist, daß insbesondere bei einem Verschleiß das Maulteil von der Aufnahme gelöst und durch ein neues Maulteil ersetzt werden kann. Hierdurch bleibt das übrige chirurgische Instrument vollständig erhalten, kann sterilisiert und immer wieder verwendet werden. Da das Maulteil nur einen äußerst geringen Anteil an dem Herstellungs- und Kostenaufwand für das chirurgische Instrument bedeutet, bewirkt die vorliegende Erfindung eine wesentliche Reduzierung der Kosten insbesondere im Krankenhauswesen.

Als Maulteil kann eine Vielzahl von Ausführungsformen in Betracht kommen. Es ist lediglich zu berücksichtigen, daß die Tätigkeit der Maulteile nicht mit ihrer lösbaren Festlegung in Konflikt kommt. D. h., mit den Maulteilen sollte keine Tätigkeit durchgeführt werden, bei der auf die Maulteile ein starker Zug ausgeübt wird. Insbesondere ist an die Ausgestaltung der Maulteile als Scherenblätter gedacht, aber auch an Maulteile, die heute bei Biopsiezangen verwendet werden.

Das übrige Instrument ist bevorzugt so ausgelegt, daß es leicht sterilisiert werden kann. Hierzu bietet sich ein chirurgisches Instrument entsprechend der P 43 32 497.5 an.

Die Maulteile können nach dem Ablösen von der Aufnahme entweder weggeworfen und durch neue ersetzt, sterilisiert und/oder nachbearbeitet werden. Ein stumpfes Scherenblatt kann beispielsweise wieder frisch geschliffen und erneut verwendet werden. Damit trägt die Erfindung wesentlich zur Verringerung des Abfalls bei, wobei zudem auch für das chirurgische Instrument und die Maulteile nur ein geringer Verpackungsanteil notwendig ist. Selbstverständlich ist es auch möglich, auf das gleiche Instrument verschieden Maulteile, mit denen unterschiedliche Tätigkeiten durchgeführt werden können, aufzusetzen.

In einem bevorzugten Ausführungsbeispiel soll das Maulteil einen Ansatz mit einem Führungsschlitz aufweisen, der mit einem Führungssteg auf einer Stützplatte der Aufnahme zusammenwirkt. Dieser Führungssteg bewirkt ein gezieltes Festlegen des Maulteiles an der Aufnahme bzw. einem entsprechenden Rastelement. Deshalb sollte an der Aufnahme auch zusätzlich noch ein Absatz vorgesehen sein, an den das Maulteil zu seiner Begrenzung anschlägt.

Zum rastenden Festlegen des Maulteils gegenüber der Aufnahme ist bevorzugt im Bereich des Führungsschlitzes eine Rastbohrung vorgesehen, welche mit einem Rastelement zusammenwirkt. Dieses Rastelement kann gesondert auf den Aufnahmen vorgesehen werden, wodurch sich allerdings die Gesamtlänge des Maules um ca. 3 mm erhöht. Da dies nicht unbedingt wünschenswert ist, bietet es sich, an, die oben erwähnte Achse auch als Rastelement zu verwenden. D.h., das Maulteil wird, geführt durch den Führungssteg in dem Führungsschlitz, so weit entlang der Aufnahme verschoben, daß der Führungsschlitz auf die Achse trifft. Durch kurzen Druck auf das Maulteil gleitet dann die Achse in die Rastbohrung ein, wobei sich der Führungsschlitz kurzzeitig etwas öffnet.

Die Aufnahme selbst besitzt dann jenseits einer Bohrung für die Achse einen Nasenansatz, welcher über einen Spreizhebel gelenkig mit einem Zug- oder Druckelement verbunden ist. Dieses Zug- oder Druckelement bewirkt die Drehbewegung der Aufnahme und damit des Maulteiles um die Achse.

In einem weiteren Ausführungsbeispiel ist am Maulteil und/oder der Aufnahme ein elastisches Element angeordnet, welches in Gebrauchslage zumindest eine Hinterschneidung, eine Randkante od.dgl. in bzw. an der Aufnahme hintergreift.

Dieses Hintergreifen des elastischen Elementes bedeutet, daß ein Lösen des Maulteils von, der Aufnahme erst dann stattfinden kann, wenn das elastische Element ausgelenkt wird, sodaß es nicht mehr die Hinterschneidung, gleich welcher Art, hintergreift. Das bedeutet aber auch, daß irgendein Hilfsmittel zur Verfügung gestellt werden muß, um ein Auslenken des elastischen Elementes zu bewirken. Ein unabsichtliches Auslenken soll vermieden sein. Damit ist die Sicherheit der Festlegung des Maulteils an der Aufnahme gewährleistet, vor allem muß der Operateur nicht mehr befürchten, daß ein Maulteil während des Eingriffs im menschlichen Körper sich von der Aufnahme löst und, beispielsweise bei endoskopischen Eingriffen, nur sehr schwierig, wieder aus dem Körper entnommen werden kann.

Für das gesicherte lösbare Festlegen des Maulteils an der Aufnahme bieten sich viele Möglichkeiten an. Dabei ist es gleichgültig, ob sich das elastische Element an dem Maulteil oder an der Aufnahme befindet. Vom Erfindungsgedanke soll immer auch die umgekehrte Anordnung umfaßt sein. Die nachfolgende Beschreibung zweier Ausführungsbeispiele ist deshalb nur beispielhaft.

In dem ersten Ausführungsbeispiel weist das Maulteil einen Ansatz auf, dem eine elastische Zunge mit einer Rastnase zugeordnet ist. Rein herstellungstechnisch bietet es sich an, diese Zunge durch Ausstanzen herzustellen. Dabei kann eine entsprechende Zunge in einem Führungsschlitz oder auch in einem C-förmigen Schlitz verbleiben.

Wesentlich ist, daß die Rastnase eine Hinterschneidung hintergreifen kann und gleichzeitig durch Auslenken der Zunge von dieser Hinterschneidung frei wird. Um dies zu erleichtern, soll die elastische Zunge einen verjüngten Bereich aufweisen, der das Auslenken erleichtert.

Als Hinterschneidung können beliebige Vorsprünge oder insbesondere auch Schlitze in der Aufnahme dienen, in welche die Rastnase einclipst. Dabei ist es wünschenswert, daß beim Einsetzen des Maulteils in die Aufnahme das Maulteil geführt wird, damit die Rastnase auch die entsprechende Hinterschneidung erreicht. Hierzu dienen entsprechende Führungsstege auf der Aufnahme, die beispielsweise mit Führungsschlitzen zusammenwirken. Dem gleichen Zweck dient aber auch eine einfache muldenförmige Einformung, welche stirnwärtig dem Maulteil bzw. einem entsprechenden Ansatz an dem Maulteil angeformt ist. Diese Einformung sucht einen kongruenten Vorsprung auf der Aufnahme.

Bei einem anderen Ausführungsbeispiel der Erfindung besteht das elastische Element im wesentlichen aus einem Raststreifen, welcher hinter eine Randkante des Führungssteges auf der Aufnahme einschrappt. Bevorzugt besitzt der Führungssteg auch beidseitig Rinnen, in denen Stege, die in die lichte Weite einer Stufenausnehmung einragen, geführt sind. Hierbei wird zum einen die Aufgabe erfüllt, daß das Maulteil beim Verbinden mit der Aufnahme geführt ist, zum anderen ergibt sich aber auch der Vorteil, daß das Maulteil auch dann nicht von der Aufnahme gelöst werden kann, wenn es seitlich von der Aufnahme abgebogen wird. Die Stege in den Rinnen verhindern ein seitliches Ausbrechen des Maulteiles, sodaß auch ein Ansetzen dieses Maulteiles an eine Aufnahme von außen her möglich ist.

Wesentliches Augenmerk ist darauf zu richten, daß die lösbare Festlegung des Maulteiles so geschieht, daß sich das Maulteil nicht versehentlich beispielsweise im Körper eines Patienten von dem übrigen chirurgischen Instrument löst. D.h., Rastbohrung und Achse müssen so aufeinander abgestimmt sein, daß zwar ein Aufschieben des Maulteiles möglich ist, daß aber ein Abziehen des Maulteiles nur unter erschwerten Bedingungen vonstatten gehen kann. Deshalb wird in einem bevorzugten Ausführungsbeispiel der Erfindung ein Montageadapter vorgesehen, welcher einen besseren Zugriff bzw. eine bessere Handhabung der Maulteile ermöglicht. Die Maulteile sind in dem Montageadapter so angeordnet, daß sie nicht verschoben werden können. Gleichzeitig ist aber eine Öffnung in dem Montageadapter vorhanden, in die der vordere Teil des chirurgischen Instrumentes ohne die Maulteile eingeführt werden kann. Da in diesem Fall der Montageadapter einen höheren Druck beim Aufklipsen der Maulteile auf die Achse erlaubt, kann beispielsweise der Durchmesser der Rastbohrung etwas geringer gehalten oder auch die Dicke des Ansatzes mit der Rastbohrung vergrößert werden. Dies wird so abgestimmt, daß ein Abziehen der Maulteile nur mit zwei Fingern nicht mehr möglich ist.

Selbstverständlich sind für einen Montageadapter viele Möglichkeiten denkbar und sollen im Rahmen der vorliegenden Erfindung liegen.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine Draufsicht auf einen vorderen Teil eines chirurgischen Instruments;
Figur 2 eine Draufsicht auf das vordere Teil des chirurgischen Instruments gemäß Fig. 1 um 90° gedreht;
Figur 3 einen Längsschnitt durch den vorderen Teil des chirurgischen Instruments gemäß Fig. 2 entlang Linie III-III;
Figur 4 eine Seitenansicht eines erfindungsgemäßen Scherenblattes;
Figur 5 eine Seitenansicht einer erfindungsgemäßen Scherenblattaufnahme;
Figur 6 eine Draufsicht auf das Scherenblatt gemäß Fig. 4;
Figur 7 eine Draufsicht auf die Scherenblattaufnahme gemäß Fig. 5;
Figur 8 einen Längsschnitt durch einen erfindungsgemäßen Montageadapter mit eingelegten Scherenblättern und ein vorderer Teil des chirurgischen Inbstruments;
Figur 9 eine Seitenansicht eines weiteren Ausführungsbeispiels eines erfindungsgmäßen Maulteils;
Figur 10 eine Draufsicht auf das Maulteil gemäß Fig. 9;
Figur 11 eine Seitenansicht einer erfindungsgemäßen Aufnahme für das Maulteil gemäß Fig. 9;
Figur 12 eine Draufsicht auf die Aufnahme gemäß Fig. 11;
Figur 13 eine Seitenansicht der erfindungsgemäßen Aufnahme gemäß Fig. 11 mit eingerastetem Maulteil gemäß Fig. 9;
Figur 14 eine Draufsicht auf zwei Maulteile in Gebrauchslage eingerastet in den entsprechenden Aufnahmen;
Figur 15 eine Seitenansicht eines weiteren Ausführungsbeispiels eines erfindungsgemäßen Maulteils;
Figur 16 eine Draufsicht auf das Maulteil gemäß Fig. 15;
Figur 17 eine Seitenansicht eines weiteren Ausführungsbeispiels eines erfindungsgemäßen Maulteiles;
Figur 18 eine Draufsicht auf die Aufnahme gemäß Fig. 17;
Figur 19 eine Seitenansicht eines weiteren Ausführungsbeispiels eines erfindungsgemäßen Maulteils;

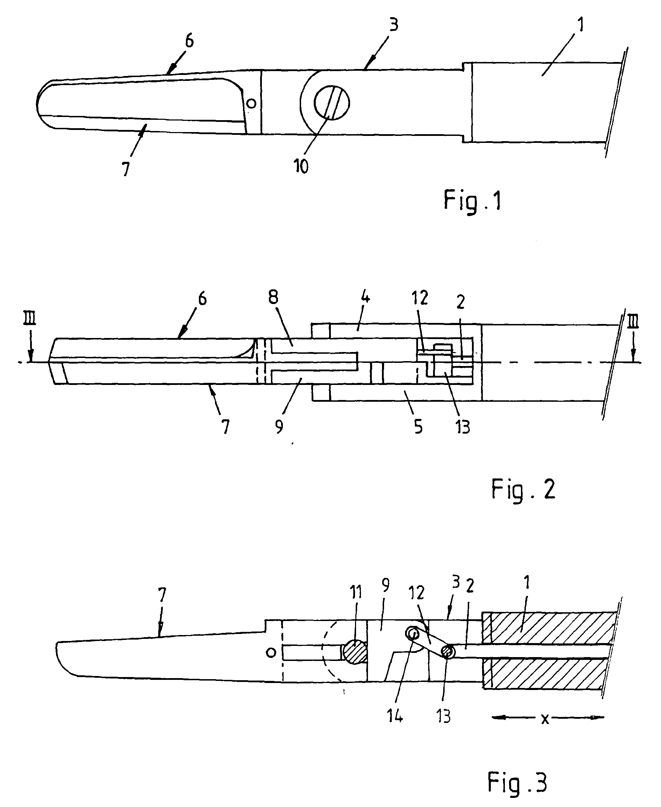

Von einem erfindungsgemäßen chirurgischen Instrument ist gemäß Fig. 1 ein vorderer Teil eines Außenrohres 1 gezeigt, in dem eine Zugstange 2 (siehe Fig. 2) gleitet. An das Außenrohr 1 schließt ein Halter 3 an, der gemäß Fig. 2 eine Gabel mit zwei Gabelschenkeln 4 und 5 ausbildet, zwischen die zwei Maulteile 6 und 7 eingesetzt sind. Im vorliegenden Ausführungsbeispiel sind beide Maulteile 6 und 7 als Scherenblätter ausgebildet.

Zur Verbindung der Maulteile 6 und 7 mit dem Halter 3 und zu der drehbaren Lagerung sind zwei Aufnahmen 8 und 9 vorgesehen, welche zusammen und mit den Gabelschenkeln 4 und 5 über eine Schlußschraube 10 verbunden sind. An die Schlußschraube 10 schließt eine in Fig. 3 gezeigte Rasterachse 11 an, um welche die Aufnahmen 8 und 9 bzw. die Maulteile 6 und 7 drehbar angeordnet sind.

Die Drehbarkeit der Aufnahmen 8 und 9 um die Rasterachse 11 wird durch ein Bewegen der Zugstange 2 in Richtung x bewirkt, wobei die Zugstange 2 über Spreizhebel, von denen nur ein Spreizhebel 12 in Fig. 3 gezeigt ist, mit den Aufnahmen 8 und 9 verbunden ist. Diese Spreizhebel 12 bilden einerseits mit der Zugstange 2 eine Gelenkachse 13 und andererseits mit der Aufnahme 9 bzw. 8 ein Drehgelenk 14. Da die Spreizhebel 12 nach außen angestellt sind, werden bei einer Bewegung der Zugstange 2 in Richtung x die Aufnahmen 8 und 9 um die Rasterachse 11 gedreht, wodurch ein Öffnen und Schließen der Maulteile 6 und 7 stattfindet. Eine ähnliche Anordnung ist in der P 43 32 497 beschrieben.

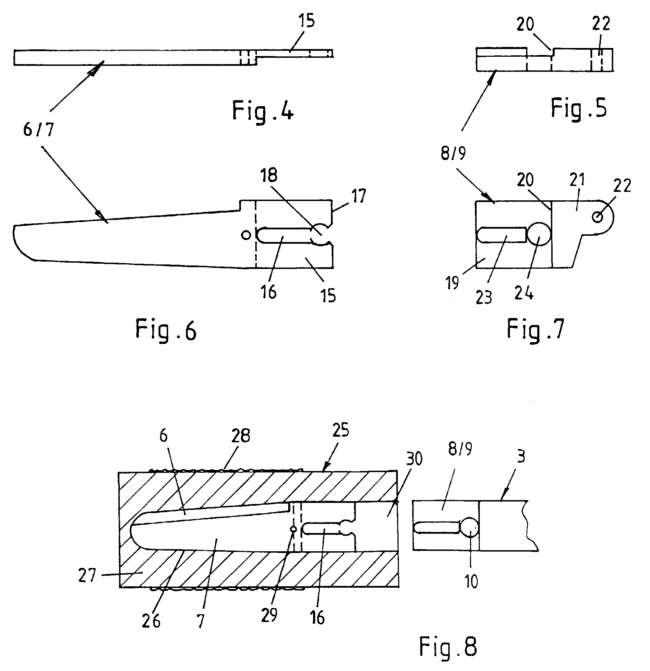

Jedes Maulteil 6 bzw. 7 weist gemäß Fig. 4 und 6 einen plättchenförmigen Ansatz 15 auf, in den ein Führungsschlitz 16 eingeformt ist. Nahe einer Stirnfläche 17 ist in den Bereich des Führungsschlitzes 16 eine Rastbohrung 18 eingebracht.

Jede Aufnahme 8 und 9 weist eine Stützplatte 19 sowie daran über einen Absatz 20 einen Nasenansatz 21 auf, welcher eine Bohrung 22 zur Aufnahme des Drehgelenkes 14 besitzt.

Auf die Stützplatte 19 ist ein Führungssteg 23 aufgesetzt, der beim Zusammenfügen von Maulteil 6/7 und Aufnahme 8/9 in den Führungsschlitz 16 eingleitet. Hierdurch wird das Maulteil 6/7 in Richtung auf eine Rasterbohrung 24 geführt, die in Gebrauchslage von der Rasterachse 11 durchsetzt ist. Durch Druck auf das Maulteil 6 bzw. 7 schnappt die Rasterachse 11 in die Rastbohrung 18 im Bereich des Führungsschlitzes 16 ein, wodurch die Maulteile 6/7 mit der Aufnahme 8/9 lösbar gekoppelt werden.

Bevorzugt befinden sich Maulteile 6 und 7 in einem Montageadapter 25, der bevorzugt so ausgestaltet ist, daß die Maulteile 6 und 7 in einer Ausnehmung 26 eines Gehäuses 27 satt anliegen. Bevorzugt ist das Gehäuse 27 auf seiner Außenfläche mit einer Riffelung 28 versehen, damit eine bessere Kraftübertragung beim Aufschieben des Montageadapters 25 auf die beiden Aufnahmen 8 und 9 erfolgt. Dabei ist der Durchmesser der Rastbohrung 18 gegenüber dem Durchmesser der Rasterachse 11 so weit verringert, daß ein Aufschieben mittels des Montageadapters 25 möglich ist, daß aber ein Abziehen der Maulteile 6/7 von den Aufnahmen 8/9 nur mittels Fingern des Operateurs oder einer anderen Bedienperson nicht möglich ist.

Zur besseren Festlegung der Maulteile 6/7 und ihrer Handhabung beim Aufschieben auf die Aufnahmen 8 und 9 sind noch Bohrungen 29 in den Maulteilen 6/7 vorgesehen, durch welche beispielsweise Stifte zur Arretierung der Maulteile 6/7 in dem Montageadapter 25 gesteckt werden können.

Zum Aufnehmen der Aufnahmen 8/9 besitzt der Montageadapter 25 im übrigen einen Führungskanal 30, der die Aufnahmen 8 und 9 zu den Maulteilen 6 und 7 hinleitet.

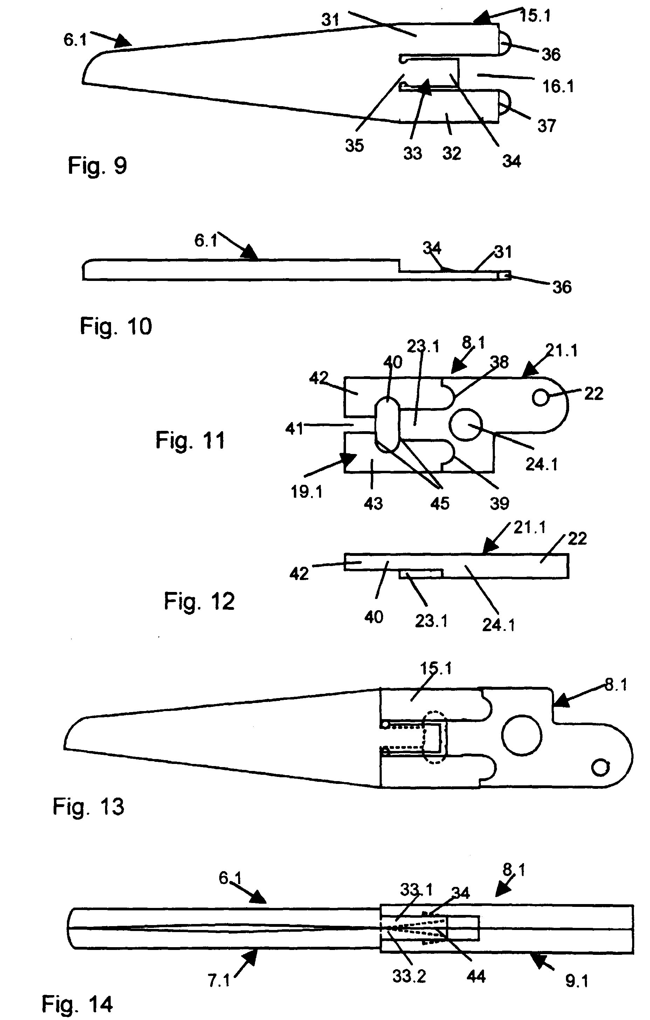

In einem weiteren Ausführungsbeispiel gemäß Fig. 9 weist ein Maulteil 6.1 einen Ansatz 15.1 auf, bei dem zwei Streifen 31 und 32 einen Führungsschlitz 16.1 ausbilden. In diesem Führungsschlitz 16.1 ist eine elastische Zunge 33 angeordnet, die eine Rastnase 34 besitzt. Ferner ist ein Hals 35 der Zunge 33 verjüngt ausgebildet, so daß ein Auslenken der Zunge 33 aus einer später beschriebenen Rastlage erleichtert ist.

Endwärtig ist jedem Streifen 31, 32 noch ein Eingriffsnocken 36 bzw. 37 angeformt, der in Gebrauchslage in entsprechende Mulden 38 und 39 einer Aufnahme 8.1 (siehe Fig. 11) eingreift. Die Aufnahme 8.1 besitzt einen breiteren Nasenansatz 21.1, in den einmal eine Bohrung 22 zur Aufnahme eines nicht näher gezeigten Gelenkteiles und zum zweiten eine Bohrung 24.1 zur Aufnahme einer Achse eingeformt ist.

An den Nasenansatz 21 schließt eine Stützplatte 19.1 an, die teilweise noch von einem Führungssteg 23.1 überragt wird, der von, dem Nasenansatz 21.1 ausgeht. Dieser Führungssteg 23.1 endet vor einem hammerkopfartigen Querschlitz 40, an den ein Längsschlitz 41 anschließt, der wiederum die Stützplatte 19 in zwei Stützstreifen 42 und 43 trennt.

Beim Einsetzen der Maulteile 6.1 und 7.1 in die entsprechenden Aufnahmen 8.1 und 9.1 gleiten die Ansätze 15.1 entlang der Stützplatte 19.1, wobei die elastischen Zungen 33.1 und 33.2 der beiden Maulteile 6.1 bzw. 7.1 zueinander ausgelenkt sind. Dies wird dadurch ermöglicht, daß die Zungen 33.1 und 33.2 keilförmig angeschnitten sind, so daß sie zwischen sich einen keilförmigen Freiraum 44 offen lassen. Beim Einschnappen in' den Querschlitz 40 hintergreifen die Rastnasen 34 entsprechende Hinterschneidungen 45, die von dem Querschlitz 40 beim Übergang in den Längsschlitz 41 gebildet werden.

Diese Anordnung gewährleistet auch die Möglichkeit einer einfachen Lösung der Maulteile 6.1 und 7.1 von den entsprechenden Aufnahmen 8.1 und 9.1. Es muß lediglich ein entsprechendes Werkzeug in den Längsschlitz 41 oder Querschlitz 40 eingeführt werden, das die elastischen Zungen 33.1 und 33.2 zueinander auslenkt, wodurch die Rastnasen 34 von den Hinterschneidungen 45 frei werden.

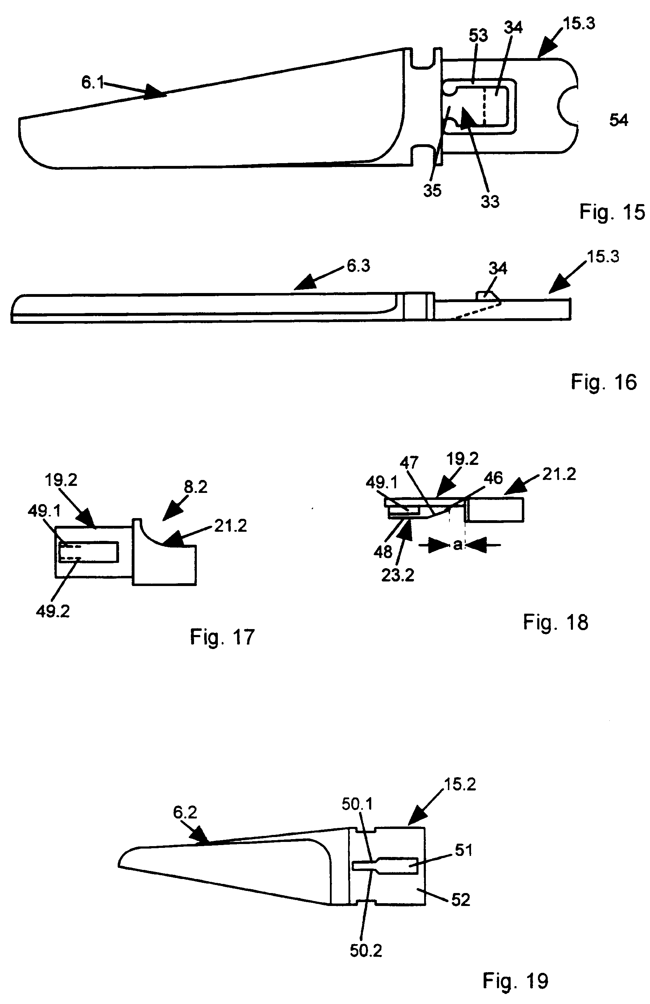

Das Ausführungsbeispiel eines Maulteils gemäß den Figuren 15 und 16 ähnelt demjenigen nach den Figuren 9 - 14. Allerdings weist hier ein Ansatz 15.3 keinen durchgehenden Führungsschlitz auf, sondern die elastische Zunge 33 ist aus dem Ansatz 15.3 durch einen C-förmigen Schlitz 53 herausgeformt.

Ferner besitzt der Ansatz 15.3 stirnwärtig eine muldenartige Einformung 54, welche mit einem entsprechenden, nicht näher gezeigten Eingriffsnocken einer dementsprechend geformten Aufnahme zusammenwirkt.

Eine Aufnahme 8.2 gemäß den Fig. 15 und 16 weist ebenfalls eine Stützplatte 19.2 auf, die an einen Nasenansatz 21.2 angeformt ist. Auf dieser Stützplatte 19.2 befindet sich ein Führungssteg 23.2, dessen hintere Randkante 46 einen Abstand a von dem Nasenansatz 21.2 einhält. Von dieser Randkante 46 steigt eine Steigfläche 47 an, die dann in einen blockartigen Ansatz 48 ausläuft. In diesen blockartigen Ansatz 48 sind von vorne her angrenzend an die Stützplatte 19.2 zwei Rinnen 49.1 und 49.2 eingeformt, in welche in Gebrauchslage Stege 50.1 bzw. 50.2 eines Maulteiles 6.2 eingreifen. Diese Stege 50.1 und 50.2 sind Teile einer Stufenausnehmung 51, die aus einem entsprechenden Ansatz 15.2 herausgeformt ist. Hierdurch wird auch am Ende des Ansatzes 15.2 ein Raststeg 52 ausgebildet. Dieser Raststeg 52 schnappt in den Bereich zwischen der Randkante 46 und dem Nasenansatz 21.2 ein und hintergreift die Randkante 46. Gleichzeitig ist das Maulteil 6.2 mit den Stegen 50.1 und 50.2 in den Rinnen 49.1 und 49.2 des Führungssteges 23 geführt, so daß auf diese Weise das Maulteil 6.2 an der Aufnahme 8.2 festgelegt ist.

Der Vorteil dieser Ausführungsform der Verbindung zwischen Maulteil 6.2 und der Aufnahme 8.2 besteht darin, daß das Maulteil 6.2 nur dann von der Aufnahme 8.2 gelöst werden kann, wenn der Raststeg 52 bzw. der Ansatz 15.2 so abgebogen wird, daß der Raststeg 52 auf die Steigfläche 47 aufgleiten kann. In diesem Fall ist es gleichgültig, ob die entsprechenden Führungsstege von benachbarten Aufnahmen zueinander oder entgegengesetzt nach außen gerichtet sind.

## Patentansprüche

1. Chirurgisches Instrument insbesondere für die Endoskopie mit einem Außenrohr (1), in dem ein Zug- oder Druckelement (2) zum Betätigen von zumindest einem Maulteil (6, 7) geführt ist, das um eine Achse (11) dreht,
dadurch gekennzeichnet,
daß das Maulteil (6, 7) über eine Aufnahme (8, 9) mit der Achse (11) verbunden und das Maulteil (6, 7) gegenüber der Aufnahme (8, 9) lösbar festgelegt ist, während andernends des Maulteils (6, 7) die Aufnahme (8, 9) gelenkig (12, 13, 14) mit dem Zug- oder Druckelement (2) verbunden ist.

2. Chirurgisches Instrument nach Anspruch 1, dadurch gekennzeichnet, daß das Maulteil (6, 7) einen Ansatz (15) mit einem Führungsschlitz (16) aufweist, der mit einem Führungssteg (23) auf einer Stützplatte (19) der Aufnahme (8, 9) zusammenwirkt.

3. Chirurgisches Instrument nach Anspruch 1 oder 2, dadurch gekennzeichnet, daß in das Maulteil (6, 7) bzw. den Ansatz (15) im Bereich des Führungsschlitzes (16) eine Rastbohrung (18) eingeformt ist.

4. Chirurgisches Instrument nach Anspruch 3, dadurch gekennzeichnet, daß der Rastbohrung (18) ein Rastelement an- bzw. auf der Aufnahme (8, 9) zugeordnet ist.

5. Chirurgisches Instrument nach Anspruch 4, dadurch gekennzeichnet, daß als Rastelement die Achse (11) dient, welche eine Bohrung (24) in der Aufnahme (8, 9) durchsetzt.

6. Chirurgisches Instrument nach wenigstens einem der Ansprüche 1 bis 5, dadurch gekennzeichnet, daß die Aufnahme (8, 9) einen Nasenansatz (21) aufweist, welcher über einen Spreizhebel (12) gelenkig mit einem Zug- oder Druckelement (2) verbunden ist.

7. Chirurgisches Instrument nach wenigstens einem der Ansprüche 1 bis 6, dadurch gekennzeichnet, daß das Maulteil (6, 7) eine Bohrung (29) zur Halterung aufweist.

8. Chirurgisches Instrument nach Anspruch 1 dadurch gekennzeichnet, daß am Maulteil (6.1, 6.2, 6.3, 7.1) und/oder der Aufnahme (8.1, 8.2, 9.1) ein elastisches Element (33, 52) angeordnet ist, welches in Gebrauchslage zumindest eine Hinterschneidung (45), eine Randkante (46) od.dgl. in bzw. an der Aufnahme (8.1, 9.1, 8.2) hintergreift.

9. Chirurgisches Instrument nach Anspruch 8, dadurch gekennzeichnet, daß das Maulteil (6.1, 7.1, 6.3) einen Ansatz (15.1, 15.3) aufweist, aus dem über einen Führungsschlitz (16.1) bzw. einen C-förmigen Schlitz (53) eine elastische Zunge mit einer Rastnase (34) herausgeformt ist.

10. Chirurgisches Instrument nach Anspruch 9, dadurch gekennzeichnet, daß die elastische Zunge (33) über einen verjüngten Hals (35) mit dem Ansatz (15.1, 15.3) bzw. dem Maulteil (6.1, 6.3, 7.1) verbunden ist.

11. Chirurgisches Instrument nach Anspruch 9 oder 10, dadurch gekennzeichnet, daß der Rastnase (34) ein Querschlitz (40) in der Aufnahme (8.1, 8.2, 9.1) zugeordnet ist, welcher die Hinterschneidung (45) ausbildet.

12. Chirurgisches Instrument nach Anspruch 11, dadurch gekennzeichnet, daß an den Querschlitz (40) ein Längsschlitz (41) anschließt.

13. Chirurgisches Instrument nach Anspruch 9, dadurch gekennzeichnet, daß das Maulteil (6.2) einen Ansatz (15.2) mit einer Stufenausnehmung (51) aufweist, der mit einem Führungssteg (23.2) auf der Aufnahme (8.2) zusammenwirkt.

14. Chirurgisches Instrument nach Anspruch 13, dadurch gekennzeichnet, daß die Stufenausnehmung (51) zumindest einen Steg (50.1, 50.2) aufweist, der in Gebrauchslage in eine Rinne (49.1, 49.2) des Führungssteges (23.2) eingefahren ist.

15. Chirurgisches Instrument nach Anspruch 14, dadurch gekennzeichnet, daß die Stufenausnehmung (51) von einem Raststeg begrenzt ist, der in Gebrauchslage eine Randkante (46) des Führungsstegs (23.2) hintergreift.

## Claims

1. Surgical instrument, more especially for endoscopy, having an outer tube (1), in which is guided a pull or push element (2) for actuating at least one jaw part (6, 7), which rotates about a pin (11), characterised in that the jaw part (6, 7) is connected to the pin (11) via a receiver (8, 9), and the jaw part (6, 7) is detachably secured relative to the receiver (8, 9) while, at the other end of the jaw part (6, 7), the receiver (8, 9) is pivotally (12, 13, 14) connected to the pull or push element (2).

2. Surgical instrument according to claim 1, characterised in that the jaw part (6, 7) has an extension (15) with a guide slot (16), which extension co-operates with a guide web (23) on a support plate (19) of the receiver (8, 9).

3. Surgical instrument according to claim 1 or 2, characterised in that a detent bore (18) is incorporated into the jaw part (6, 7) or respectively the extension (15) in the region of the guide slot (16).

4. Surgical instrument according to claim 3, characterised in that a locking element is associated with the detent bore (18) at or respectively on the receiver.

5. Surgical instrument according to claim 4, characterised in that the pin (11), which traverses a bore (24) in the receiver (8, 9), serves as the locking element.

6. Surgical instrument according to at least one of claims 1 to 5, characterised in that the receiver (8, 9) has a projecting extension (21) which is pivotally connected to a pull or push element (2) via a toggle lever (12).

7. Surgical instrument according to at least one of claims 1 to 6, characterised in that the jaw part (6, 7) has a bore (29) for retention purposes.

8. Surgical instrument according to claim 1, characterised in that a resilient element (33, 52) is disposed on the jaw part (6.1, 6.2, 6.3, 7.1) and/or the receiver (8.1, 8.2, 9.1), which resilient element engages, in the position of use, behind at least one undercut (45), one lateral edge (46) or the like, in or respectively at the receiver (8.1, 9.1, 8.2).

9. Surgical instrument according to claim 8, characterised in that the jaw part (6.1, 7.1, 6.3) has an extension (15.1, 15.3), out of which is fashioned, via a guide slot (16.1) or respectively a C-shaped slot (53), a resilient tongue having a detent projection (34).

10. Surgical instrument according to claim 9, characterised in that the resilient tongue (33) is connected to the extension (15.1, 15.3) or respectively to the jaw part (6.1, 6.3, 7.1) via a tapered neck (35).

11. Surgical instrument according to claim 9 or 10, characterised in that a transverse slot (40) in the receiver (8.1, 8.2, 9.1) is associated with the detent projection (34), which slot forms the undercut (45).

12. Surgical instrument according to claim 11, characterised in that a longitudinal slot (41) communicates with the transverse slot (40).

13. Surgical instrument according to claim 9, characterised in that the jaw part (6.2) has an extension (15.2) with a stepped recess (51), which extension co-operates with a guide web (23.2) on the receiver (8.2).

14. Surgical instrument according to claim 13, characterised in that the stepped recess (51) has at least one web (50.1, 50.2) which, in the position of use, is inserted into a groove (49.1, 49.2) of the guide web (23.2).

15. Surgical instrument according to claim 14, characterised in that the stepped recess (51) is defined by a detent web which, in the position of use, engages behind a lateral edge (46) of the guide web (23.2).

## Revendications

1. Instrument chirurgical, en particulier pour l'endoscopie, comprenant un tube extérieur (1) à l'intérieur duquel est guidé un élément de traction ou de poussée (2), aux fins d'actionner au moins un élément formant mâchoire (6, 7), qui tourne autour d'un axe (11),
caractérisé en ce que
l'élément formant mâchoire est lié à l'axe (11) par un support (8, 9) et que l'élément formant mâchoire (6, 7) est fixé de manière séparable au support (8, 9), tandis qu'à l'extrémité du support (8, 9) éloignée de l'élément formant mâchoire (6, 7) est liée de manière articulée (12, 13, 14) à l'élément de traction ou de poussée (2).

2. Instrument chirurgical selon la revendication 1, caractérisé en ce que l'élément formant mâchoire (6, 7) comporte un embout (15) avec une fente de guidage (16) qui coopère avec une nervure de guidage (23) sur une plaquette d'appui (19) du support (8, 9).

3. Instrument chirurgical selon la revendication 1 ou 2, caractérisé en ce qu'un trou d'encliquetage (18) est aménagé dans l'élément formant mâchoire (6, 7) ou dans l'embout (15), dans la région de la fente de guidage (16).

4. Instrument chirurgical selon la revendication 3, caractérisé en ce qu'un élément d'encliquetage de ou sur le support (8, 9) est associé au trou d'encliquetage (18).

5. Instrument chirurgical selon la revendication 4, caractérisé en ce que l'axe (11) qui traverse un trou (24) dans le support (8, 9) sert d'élément d'encliquetage.

6. Instrument chirurgical selon au moins une des revendications 1 à 5, caractérisé en ce que le support (8, 9) comporte une saillie (21) qui est liée de manière articulée à un élément de traction ou de poussée (2) par l'intermédiaire d'un levier d'ouverture (12).

7. Instrument chirurgical selon au moins une des revendications 1 à 6, caractérisé en ce que l'élément formant mâchoire (6, 7) comporte un trou (29) de fixation.

8. Instrument chirurgical selon la revendication 1, caractérisé en ce qu'un élément élastique (33, 52) est disposé sur l'élément formant mâchoire (6.1, 6.2, 6.3, 7.1) et/ou sur le support (8.1, 8.2, 9.1), lequel élément élastique, dans la position d'utilisation, s'accroche dans au moins une contre-dépouille (45), une arête de bord (46) ou similaire dans ou sur le support (8.1, 8.2, 9.1).

9. Instrument chirurgical selon la revendication 8, caractérisé en ce que l'élément formant mâchoire (6.1, 6.2, 6.3, 7.1) comporte un embout (15.1, 15.3) dans lequel une languette élastique avec un nez d'encliquetage (34) est définie par une fente de guidage (16.1) ou une fente en forme de C (53).

10. Instrument chirurgical selon la revendication 9, caractérisé en ce que la languette élastique (33) est liée à l'embout (15.1, 15.3) ou à l'élément formant mâchoire (6.1, 6.3, 7.1) par l'intermédiaire d'un col (35) resserré,

11. Instrument chirurgical selon la revendication 9 ou 10, caractérisé en ce qu'une fente transversale (40) dans le support (8.1, 8.2, 9.1) qui forme la contre-dépouille (45) est associée au nez d'encliquetage (34).

12. Instrument chirurgical selon la revendication 11, caractérisé en ce qu'une fente longitudinale (41) se raccorde à la fente transversale (40).

13. Instrument chirurgical selon la revendication 9, caractérisé en ce que l'élément formant mâchoire (6.2) comporte un embout (15.2) avec un évidement étagé (51) qui coopère avec une nervure de guidage (23.2) sur le support (8.2).

14. Instrument chirurgical selon la revendication 13, caractérisé en ce que l'évidement étagé (51) comporte au moins une barrette (50.1, 50.2) qui, dans la position d'utilisation, est rentrée dans un sillon (49.1, 49.2) de la nervure de guidage (23.2).

15. Instrument chirurgical selon la revendication 14, caractérisé en ce que l'évidemment étagé est limité par un bourrelet d'encliquetage qui, dans la position d'utilisation, s'engage derrière une arête de bord (46) de la nervure de guidage (23.2).
